# EUROPÄISCHE PATENTANMELDUNG

(11) **EP 0 788 806 A2**
(43) Veröffentlichungstag der Anmeldung: **13.08.1997**
(21) Anmeldenummer: 97100426.2
(22) Anmeldetag: 13.01.1997
(51) Int. Cl.: A61M 16/10, A62B 21/00

(54) **Mittel zur Freisetzung von Sauerstoff in Sauerstoffgeneratoren**

(30) Priorität: 22.01.1996 DE 19602149
(71) Anmelder: Canavate Riera, Gertraud, 83395 Freilassing (DE)
(72) Erfinder: Canavate Riera, Gertraud, 83395 Freilassing (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(57) **Zusammenfassung**

Mittel zur Freisetzung von Sauerstoff in Sauerstoffgeneratoren, das Natriumcarbonat-Peroxyhydrat und eine Natriumsulfat/Katalase-Mischung enthält. Das Natriumcarbonat-Peroxyhydrat und die Natriumsulfat/Katalase-Mischung sind bis zur Verwendung im Sauerstoffgenerator getrennt voneinander gehalten. Zur Freisetzung des Sauerstoffs werden sie im Sauerstoffgenerator mit Wasser in Kontakt gebracht.

## Beschreibung

Die Erfindung betrifft ein Mittel zur Freisetzung von Sauerstoff in Sauerstoffgeneratoren. Sauerstoffgeneratoren werden zu medizinischen Zwecken als Inhalationsgeräte eingesetzt.
In ihnen werden Sauerstoff freisetzende Substanzen mit Wasser in Kontakt gebracht.

Ein Mittel enthält als Sauerstoff liefernde Substanz pulverförmiges Natriumcarbonat-Peroxyhydrat (2 Na₂CO₃ · 3 H₂O₂) zusammen mit Natriumsulfat und Natriumchlorid enthaltenden Tabletten in einer Verpackungseinheit. Wird dieses Mittel mit Wasser in Kontakt gebracht, so ergibt sich folgender zeitlicher Verlauf der Sauerstoffentwicklung. Nach einer Verzögerungsphase von etwa 10 Minuten setzt die Sauerstoffentwicklung ein, erreicht nach 25 bis 30 Minuten ein Maximum von etwa 0,65 l/min (∼ 40 l/h) und fällt innerhalb weiterer 10 Minuten auf etwa 0,15 l/min (∼ 10 l/h) ab.

Katalase (Wasserstoffperoxid: Wasserstoffperoxid-Oxidoreductase, EC 1.11.1.6) ist ein Enzym, das in pflanzlichen und tierischen Geweben verbreitet ist und Wasserstoffperoxid in Wasser und Sauerstoff zerlegt. Katalase ist ein tetrameres Eisenprotein vom Molekulargewicht 240000 bis 245000 mit 4 Häm-Molekülen in Form von Ferriprotoporphyrin mit Eisen (III).

Aufgabe der vorliegenden Erfindung war es, ein Mittel zur Freisetzung von Sauerstoff in Sauerstoffgeneratoren zur schaffen, das eine Sauerstoffentwicklung ohne längere Verzögerungsphase mit einem möglichst langsamen Abfall der Sauerstoffentwicklung vom Beginn bis zum Ende ergibt. Das Maximum der Sauerstoffentwicklung sollte zu Beginn über 1 l/min und bevorzugt über 2 l/min liegen. In einer weiteren bevorzugten Ausführungsform sollte die Sauerstoffentwicklung über einen Zeitraum von 20 min anhalten.

Zur Lösung dieser Aufgabe wird ein Mittel vorgeschlagen, das Natriumcarbonat-Peroxyhydrat und eine Natriumsulfat/Katalase-Mischung enthält, wobei das Natriumcarbonat-Peroxyhydrat und die Natriumsulfat/Katalase-Mischung bis zur Freisetzung des Sauerstoffs in Wasser getrennt voneinander gehalten sind.

Wesentlich ist, daß das Natriumcarbonat-Peroxycarbonat und die Natriumsulfat/Katalase-Mischung bis zur Anwendung im Sauerstoffgenerator nicht in Kontakt miteinander kommen. Es empfiehlt sich, beide getrennt in jeweils reißfesten Kunststoffbeuteln, z.B. aus Polyethylen, bis zur Anwendung verpackt zu lagern. Erst beim Einsatz im Sauerstoffgenerator sind sie miteinander in Wasser in Kontakt zu bringen. Durch die getrennte Aufbewahrung vor der Anwendung wird ein Verlust an aktivem Sauerstoff vermieden. Empfehlenswert ist eine kühle und trockene Lagerung.

Das Natriumcarbonat-Peroxyhydrat (2Na₂CO₃ · 3H₂O₂) wird vorzugsweise in pulverförmiger Form verwendet. In dieser Form ist es als Handelsprodukt z.B. von der Firma Solvay Interox GmbH unter dem Warenzeichen Oxyper mit einem Aktivsauerstoffgehalt von mindestens 13,5 % zu erhalten.

Die Natriumsulfat/Katalase-Mischung wird aus wasserfreiem Natriumsulfat, reinst DAB und Katalase durch Mischen hergestellt. Natriumsulfat, wasserfrei reinst DAB in Lebensmittelqualität ist z.B. ein Handelsprodukt der Merck KGaA.
Katalase EC 1.11.1.6 Hydrogenperoxid-Oxidoreductase ist ebenfalls als Handelsprodukt erhältlich, z.B. von Solvay Enzymes GmbH & Co. KG unter dem Warenzeichen Solvay Microcatalase-L 240 in flüssiger Form. Die Mischung kann als solche in Kunststoffbeutel abgefüllt werden oder tablettiert und dann in Tablettenform verpackt werden. Das Natriumsulfat dient als Trägermaterial für die Katalase.

Vorzugsweise wird der Natriumsulfat/Katalase-Mischung noch ein Entschäumungsmittel, z.B. ein Siliconentschäumer, in geringer Menge zugesetzt. Solche Entschäumungsmittel sind ebenfalls im Handel erhältlich, z.B. Siliconentschäumer LAB der Firma Merck KGaA.

Natriumsulfat und Katalase werden vorzugsweise in einem Gewichtsverhältnis von 4:1 bis 15:1 und besonders bevorzugt von 6:1 bis 11:1 miteinander gemischt.

Das Natriumcarbonat-Peroxyhydrat und die Natriumsulfat/Katalase-Mischung werden zur Sauerstofferzeugung vorzugsweise in einem Gewichtsverhältnis von 25:1 bis 45:1 und besonders bevorzugt von 30:1 bis 40:1 verwendet.

Für die Sauerstofferzeugung werden in der Regel Verpackungseinheiten des Natriumcarbonat-Peroxyhydrats von mindestens 100 g und vorzugsweise 150 bis 200 g sowie Verpackungseinheiten der Natriumsulfat/Katalase-Mischung von 5 bis 6 g verwendet.
Die Erfindung wird im folgenden an Hand der Beispiele und der Figuren näher erläutert:
- Fig. 1: zeigt den zeitlichen Verlauf der Sauerstofferzeugung eines bekannten Mittels;
- Fig. 2: zeigt den zeitlichen Verlauf der Sauerstofferzeugung eines erfindungsgemäßen Mittels;
- Fig. 3: zeigt den zeitlichen Verlauf der Sauerstofferzeugung eines bevorzugten erfindungsgemäßen Mittels.

Die erzeugte Sauerstoffmenge wurde jeweils mit Hife eines Rotameters gemessen.

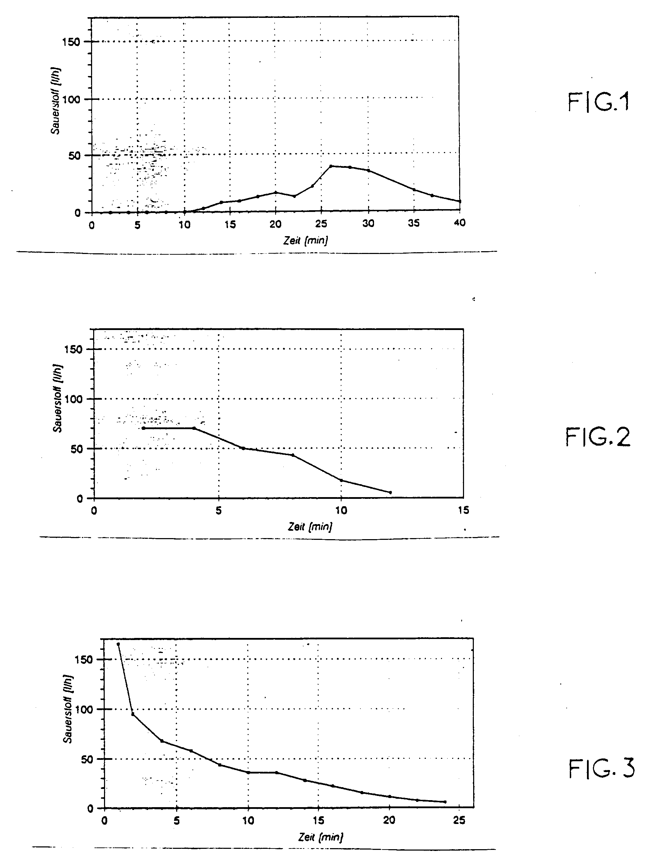

Ein bekanntes Mittel weist einen Beutelinhalt von ca. 100 g Natriumcarbonat-Peroxyhydrat und zwei Tabletten à 0,9 bis 1,0 g auf, die Natriumsulfat und Natriumchlorid enthalten.
Bei Kontakt des Beutelinhalts mit Wasser erhält man eine Sauerstofferzeugung, deren zeitlicher Verlauf in Fig. 1 dargestellt ist. Dabei ist die je Zeiteinheit freigesetzte Sauerstoffmenge (l/h) gegen die Zeit (min) aufgetragen.
Die Sauerstofferzeugung setzt nach etwa 10 min ein, erreicht nach 25 bis 30 min ein Maximum von etwa 40 l/h und fällt innerhalb weiterer 10 min auf etwa 10 l/h ab.

### Beispiel 1

Es werden 100 g Natriumcarbonat-Peroxyhydrat (Oxyper^{R} der Firma Solvay Interox) verwendet. Eine Mischung von 5 g Natriumsulfat (wasserfrei, reinst DAB, Lebensmittelqualität der Firma Merck KGaA), 500 µl 1.11.1.6 Hydroxyperoxid-Oxidoreductase (Microcatalase-L 240 der Firma Solvay Enzymes GmbH & Co. KG) und 100 µl Siliconentschäumer (LAB der Firma Merck KGaA) wird hergestellt. Das Natriumcarbonat-Peroxyhydrat und die Mischung wurden vor der Verwendung nicht zusammen gelagert. Bei Zugabe des Natriumcarbonat-Peroxyhydrats und der Mischung (kein Preßling)zu Wasser erhält man den in Fig. 2 dargestellten zeitlichen Verlauf der Sauerstofffreisetzung. Ein Vergleich der Fig. 1 und 2 zeigt, daß die Sauerstofffreisetzung wesentlich schneller einsetzt und von einem anfänglichen Maximum von etwa 70 l/h innerhalb von etwa 10 min auf 20 l/h abfällt.

### Beispiel 2

In diesem Beispiel wurde in derselben Weise und unter Verwendung derselben Materialien wie in Beispiel 1 vorgegangen mit der Ausnahme, daß die Menge des Natriumcarbonat-Peroxyhydrats auf 200 g erhöht wurde. Der Verlauf der Sauerstofffreisetzung ist in Fig. 3 dargestellt. Die Sauerstofferzeugung setzt sofort nach Zugabe des Natriumcarbonat-Peroxyhydrats und der Natriumsulfat/Katalase/Entschäumer-Mischung zum Wasser ein. Nach einem anfänglichen steileren Abfall innerhalb von etwa 4 min erniedrigt sich die Sauerstoffentwicklung pro Zeiteinheit allmählich während eines Zeitraums von etwa 20 min.

## Patentansprüche

1. Mittel zur Freisetzung von Sauerstoff in Sauerstoffgeneratoren, das Natriumcarbonat-Peroxyhydrat und eine Natriumsulfat/Katalase-Mischung enthält, wobei die Natriumsulfat/Katalase-Mischung und das Natriumcarbonat-Peroxyhydrat bis zur Freisetzung des Sauerstoffs in Wasser getrennt voneinander gehalten sind.

2. Mittel nach Anspruch 1, bei dem das Natriumcarbonat-Peroxyhydrat pulverförmig vorliegt.

3. Mittel nach Anspruch 1 oder 2, bei dem die Natriumsulfat/Katalase-Mischung in ungepreßter Form vorliegt.

4. Mittel nach einem der Ansprüche 1 bis 3, bei dem die Natriumsulfat/Katalase-Mischung ein Entschäumungsmittel enthält.

5. Mittel nach einem der Ansprüche 1 bis 4, bei dem das Natriumcarbonat-Peroxyhydrat und die Natriumsulfat/Katalase-Mischung jeweils bis zur Sauerstofffreisetzung in getrennten Kunstoffbeuteln enthalten sind.

6. Mittel nach einem der Ansprüche 1 bis 5, bei dem das Gewichtsverhältnis von Natriumcarbonat-Peroxyhydrat zu der Natriumsulfat/Katalase-Mischung im Bereich von 25:1 bis 45:1 liegt.

7. Mittel nach Anspruch 6, bei dem das Gewichtsverhältnis im Bereich von 30:1 bis 40:1 liegt.

8. Mittel nach einem der Ansprüche 1 bis 7, bei dem das Gewichtsverhältnis von Natriumsulfat zu Katalase in der Mischung im Bereich von 4:1 bis 15:1 liegt.

9. Mittel nach Anspruch 8, bei dem das Gewichtsverhältnis im Bereich von 6:1 bis 11:1 liegt.
